# EUROPEAN PATENT APPLICATION

(11) **EP 4 450 631 A1**
(43) Date of publication of application: **23.10.2024**
(21) Application number: 22906280.7
(22) Date of filing: 02.12.2022
(51) Int. Cl.: C12P 7/26, C12N 9/02, C12N 15/53, C12N 15/81, C12N 1/19, C12R 1/865

(54) **USE OF OXIDOREDUCTASE AND MUTANT THEREOF IN BIOSYNTHESIS OF NOOTKATONE**

(30) Priority: 13.12.2021 CN 202111517500
(71) Applicant: Terpiot (Guangzhou) Biotechnology Co., Ltd., Guangzhou, Guangdong 510000 (CN)
(72) Inventor: LI, Shuang, Guangzhou, Guangdong 510640 (CN); CHEN, Dongying, Guangzhou, Guangdong 510640 (CN); LI, Wen, Guangzhou, Guangdong 510640 (CN); ZHU, Chaoyi, Guangzhou, Guangdong 510640 (CN)
(74) Representative: Schulz Junghans Patentanwälte PartGmbB
(86) International application number: PCT/CN2022/136155
(87) International publication number: WO 2023/109530

(57) **Abstract**

Provided is the use of oxidoreductase and a mutant thereof in the biosynthesis of nootkatone. The oxidoreductase is derived from *Wickerhamomyces anomalus* M15 and has a high ability to convert nootkatol, and an oxidoreductase mutant having a higher ability is obtained by means of site-directed mutation. Compared with the existing oxidoreductase that is capable of catalyzing nootkatol, the oxidoreductase and the mutant thereof have a good substrate tolerance, a high conversion rate and a high salt tolerance, provide conditions for the synthesis of nootkatone using an enzyme catalytic method in vitro, and achieve the synthesis of nootkatone by means of catalyzing nootkatol in an environmentally-friendly and efficient manner.

## Description

### Field of the Invention

The present disclosure belongs to the technical field of bioengineering, and particularly relates to the use of oxidoreductase and a mutant thereof in biosynthesis of nootkatone.

### Background of the Invention

As a sesquiterpene compound with pomelo aroma and a slightly bitter taste, nootkatone is first extracted from cedars in Alaska and exists in plants such as grapefruits and citruses. Pure nootkatone is a white to yellowish crystal, and is approved by FDA and EPA for the preparation of edible and tobacco flavors of grapefruits, oranges, tropical fruits, etc. Nootkatone, which exists in a spray form, is an effective insecticide against lone star ticks and deer ticks. In addition, it also has a good killing effect on mosquitoes, bedbugs, lice, etc. Due to its good volatility and non-toxicity to humans, nootkatone is considered to be an environmentally friendly insecticide. In 2014, CDC officially approved two companies to produce nootkatone insecticides. In addition, in recent years, research has found that nootkatone has an effect on inhibiting proliferation of cancer cells and has potential therapeutic effects on neuroinflammation and Alzheimer's disease. Research and development of nootkatone have aroused great interest in the pharmaceutical industry, so an application prospect of nootkatone is huge.

As one of sesquiterpene compounds, nootkatone has a complex chemical structure, which hinders its industrial mass production. At present, there are three main methods for producing nootkatone: a physical extraction method, a chemical synthesis method and a biocatalytic conversion method. Nootkatone separated from the grapefruits as raw materials by distillation, extraction and other processes has the problems of a low effective concentration, complex procedures such as separation and purification, being easily affected by seasons and climate changes, etc., and thus cannot meet industrial demands. At present, the most commonly used method in the industry is chemical synthesis of nootkatone from valencene (a relatively cheap precursor); however, catalysts (such as chromium trioxide, cobalt acetylpyruvate or other heavy metal salts) used in oxidizing reactions produce more toxic wastes, which is contrary to a concept of green development. In order to meet the increasing market demand on nootkatone, the use of a biocatalytic conversion method is not limited by raw materials, can avoid the problems of high energy consumption, low yield, environmental pollution, etc. during plant extraction, and thus has great advantages.

In recent years, a great progress has been made in metabolic engineering of microorganisms. Construction of an efficient microbial cell factory and improvement on its physiological performance are expected to efficiently convert cheap raw material substrates to high value-added target products, which greatly lowers the production cost of microbial fermentation. Construction of heterologous expression of related enzymes in the microorganisms such as a yeast and *Escherichia coli* to produce nootkatone has attracted much attention. Researchers have isolated a valencene synthase gene from *C. sinensis.* The valencene synthase gene can effectively perform functional expression in the *Escherichia coli,* and can also be used for subsequent production of nootkatone (Chappell J.et al. Novel sesquiterpene synthase gene and protein: US, US20120196340 A1 [P]. 2006.). Valencene dioxygenase (ValOx) derived from *P. sapidus* is further found to be expressed in cytoplasm of the *Escherichia coli,* and to convert the valencene to nootkatol and nootkatone by hydrogen peroxide as an intermediate (Zelena K. et al. Functional expression of a valencene dioxygenase from Pleurotussapidus in E. coli [J]. Bio Tec, 2012, 108:231-239.). As one of the most commonly used gene expression hosts, a model organism yeast is simple in culture conditions, clear in genetic background, and liable to perform genetic modification, where marine yeasts are obviously more tolerant to the presence of inhibitory compounds such as acetic acid, formic acid, furfural, vanillin and salts than terrestrial yeasts. The most tolerant *Wickerhamomyces anomalus* is *Wickerhamomyces anomalus* M15, and its salt tolerance is 1.6 times that of *Saccharomyces cerevisiae* NCYC2592 (Greetham D. et al. Exploring the tolerance of marine yeast to inhibitory compounds for improving bioethanol production [J]. Sustainable Energy & Fuels, 2019, 3(3).). Therefore, further obtaining efficient and environmentally tolerant nootkatol dehydrogenase by genetic engineering is still urgent in the art.

NCBI includes a hypothetical protein derived from *Wickerhamomyces anomalus* NRRL Y-366-8, which has an original name of WICANDRAFT_92107 (NCBI accession number: XP_019039214.1), and includes a total of 264 amino acids; and a nucleotide sequence of a coding gene (NCBI accession number: XM_019186339.1) of the hypothetical protein includes a total of 795 nucleotides. So far, there is no literature reporting any actual function and application of the protein.

### Summary of the Invention

In order to overcome the disadvantages and the deficiencies in the prior art, an objective of the present disclosure is to provide the use of oxidoreductase and a mutant thereof in biosynthesis of nootkatone, aiming to solve the problems of low tolerable substrate concentration, low conversion rate, poor salt tolerance and non-ideal industrial production of existing alcohol dehydrogenase during biosynthesis of nootkatone.

The oxidoreductase is derived from *Wickerhamomyces anomalus* M15 and has a high ability to convert nootkatol, and an oxidoreductase mutant having a higher ability is obtained by site-directed mutation.

The objective of the present disclosure is implemented by the following technical solution:
the present disclosure provides the use of oxidoreductase and/or a mutant thereof in biosynthesis of nootkatone.

In the present disclosure, the oxidoreductase is derived from NRRL of *Wickerhamomyces anomalus* M15, having an amino acid sequence shown as SEQ ID No. 2, and including a total of 264 amino acids.

In the present disclosure, a nucleotide sequence of the oxidoreductase NRRL coding gene is shown as SEQ ID No. 1, and includes a total of 795 nucleotides.

Further, a nucleotide sequence of the oxidoreductase NRRL coding gene after being optimized by a codon is shown as SEQ ID No. 3.

The present disclosure further relates to an oxidoreductase mutant, having an amino acid sequence obtained by mutating one or two sites of the 69ₜₕ and 136ₜₕ amino acids of SEQ ID No. 2. Further, the 69ₜₕ amino acid is mutated from lysine K to threonine T, and the 136ₜₕ amino acid is mutated from glycine Gto arginine R or alanine A.

More further, the amino acid sequence of the oxidoreductase mutant is obtained by mutation of the 69ₜₕ amino acid of SEQ ID No. 2 from the lysine K to the threonine T and the 136ₜₕ amino acid of SEQ ID No. 2from the glycine G to the arginine R. The specific amino acid sequence is shown as SEQ ID No. 4.

Preferably, in the oxidoreductase mutant, the sequence of a gene coding the amino acid sequence shown as SEQ ID No. 2 is shown as SEQ ID No. 3.

In another aspect of the present disclosure, an amino acid sequence is obtained by substitution, deletion or addition of one or more amino acids in the amino acid sequence shown as SEQ ID No. 2, and sequences that maintain a homology of only 90% are also within the scope of protection of the present disclosure.

The present disclosure provides a recombinant expression vector containing the coding gene for the oxidoreductase and the mutant thereof. Preferably, the recombinant vector is the recombinant expression vector obtained by recombination of the oxidoreductase and the mutant thereof with a 2µ-type high-copy-number plasmid YEp352.

The present disclosure provides a recombinant expression cell for the recombinant expression vector containing the coding gene for the oxidoreductase and the mutant thereof. Preferably, the recombinant expression cell is obtained by transforming the recombinant expression vector obtained by recombination of the oxidoreductase and the mutant thereof with the vector into a host cell. In the present disclosure, the host cell is preferably *Saccharomyces cerevisiae,* and more preferably a *Saccharomyces cerevisiae* CEN. PK2-1 Ca strain.

The present disclosure further relates to a method for preparing nootkatone.

In the present disclosure, two methods of using the recombinant expression cell for the recombinant expression vector containing the coding gene for the oxidoreductase and the mutant thereof to achieve biosynthesis of nootkatone are provided.

One method is a whole-cell in vitro catalytic method, which is to obtain nootkatone by exogenously adding nootkatol as a precursor substance and using the recombinant expression cell of the present disclosure for in vitro transformation. A reaction substrate is the nootkatol.

The conversion efficiency of the present disclosure is expressed as a ratio of the nootkatol as the precursor substance to nootkatone as a target product within a certain period of time.

The other method, nootkatone is prepared by biological fermentation (i.e. by culturing a recombinant expression cell expressing a related enzyme in a reactor containing a medium).

In one preferred embodiment, the host cell is a eukaryotic cell, especially selected from a *Saccharomyces cerevisiae* CEN. PK2-1 Ca strain. Using the technology described in the Patent with Application No. 201910271558.6, the host cell has been genetically modified, including knocking out a limiting factor rox1 for a mevalonate pathway and down-regulating expression intensity of a downstream branch pathway-related enzyme erg9 of a precursor FPP for sesquiterpenoids, so as to increase a supply of the precursor FPP. Valencene synthase ValC derived from *Chamaecyparis nootkatensis* described in International Patent with Application No. PCT/NL2010/050848 is used to convert FPP into valencene as the precursor substance for nootkatone. Subsequently, cytochrome P450 monooxygenase (CYP450) HPO derived from *Hyoscyamus muticus* described in the Patent WO 2006/079020 is used. A cytochrome reductase AtCPR (Urban, P., et al., Cloning, yeast expression, and characterization of the coupling of two distantly related Arabidopsis thaliana NADPH-cytochrome P450 reductases with P450 CYP73A5. J. Biol. Chem., 1997, 272, 19176-19186.) from *Arabidopsis thaliana* has been described by Urban, Pd et al. in a paper in 1997, and is used to further oxidize the valencenes into the nootkatol. Furthermore, the oxidoreductase or the mutant thereof described in the present disclosure is used to convert the nootkatol to nootkatone as a final desired product.

The present disclosure also protects the use of the oxidoreductase and the mutant thereof as a nootkatol dehydrogenase with high salt tolerance in biosynthesis of nootkatone.

Compared with the prior art, the present disclosure has the following advantages and effects that:
(1) The present disclosure provides oxidoreductase NRRL derived from the *Wickerhamomyces anomalus* M15, which is the first discovered *Wickerhamomyces anomalus*-derived oxidoreductase using the nootkatol as a substrate. Meanwhile, the present disclosure has also disclosed the use of the oxidoreductase NRRL and the mutant thereof in catalyzing the nootkatol to synthesize nootkatone for the first time at home and abroad.
(2) The present disclosure provides the conditions for synthesis of nootkatone by an enzyme catalytic method in vitro, so as to greenly and efficiently catalyze the nootkatol to synthesize the corresponding nootkatone. Therefore, the defects of low yield, low efficiency, cumbersome operations, serious environmental pollution and high cost when nootkatone is obtained by physical extraction or chemical synthesis are overcome.
(3) Compared with the existing oxidoreductase that can catalyze the nootkatol, the NRRL and the mutant thereof have better substrate tolerance, higher conversion rate and higher salt tolerance. Compared with the discovered ABA2 (NCBI accession number: HM036684.1) derived from citrus, the nootkatol dehydrogenase has a significantly better effect. Results show that under the same conditions, a substrate target conversion rate obtained by using the oxidoreductase of the present disclosure is higher, being nearly 3.8 times that of ABA2 of the citrus. In addition, under the same conditions, the conversion efficiency to the nootkatol by some of mutants of the nootkatol dehydrogenase provided by the present disclosure can be nearly 2.4 times that of the oxidoreductase shown as SEQ ID No. 2 in a sequence table, so that some of the mutants of the nootkatol dehydrogenase have better substrate tolerance, and are improved in tolerance in NaCl of different concentrations (6%, 9%, 12%, 15%, 18% (W/V)).
(4) The present disclosure will provide an important tool enzyme for synthesis of nootkatone, which will bring a huge economic benefit to the synthesis industry of nootkatone.

### Brief Description of the Drawings

FIG. 1 is a schematic diagram of plasmid construction and reaction.
FIG. 2 is a diagram of gas phase detection results of whole-cell catalysis with related alcohol dehydrogenase.
FIG. 3 is a GC-MS mass spectrogram of nootkatone.
FIG. 4 is a diagram showing comparison on catalytic performance of an expression strain of an NRRL gene sequence before and after being optimized by a codon.
FIG. 5 is a diagram showing a substrate conversion efficiency of an oxidoreductase mutant.
FIG. 6 is a diagram showing effects of NaCl of different concentrations (W/V) on a conversion rate of oxidoreductase or an oxidoreductase mutant.

### Detailed Description of the Embodiments

The present disclosure is further described by combining the embodiments and the accompanying drawings, but the implementations of the present disclosure are not limited thereto.

The following embodiments are used for describing the present disclosure merely rather than limiting the scope of the present disclosure. It should be noted that those skilled in the art can make several changes and improvements without departing from the idea of the present disclosure, for example, changes on types of expression vectors, changes on method for constructing the expression vectors, changes on types of host cells, etc. The above are all within the scope of protection of the present disclosure.

*S. cerevisiae* CEN. PK2-1 Ca and *S. c*. PK2-M used in the embodiments were disclosed in "CN201910271558*-EngineeringBacterium for Saccharomyces Cerevisiae for Producing Valencene and Construction Method and Application Thereof".*

*Wickerhamomyces anomalus* M15 in the embodiments were disclosed in the literature "Greetham D. et al. Exploring the tolerance of marine yeast to inhibitory compounds for improving bioethanol production [J]. Sustainable Energy & Fuels, 2019, 3(3).".

### Example 1: Cloning of Oxidoreductase NRRL and Construction of Expression Vector

*Wickerhamomyces anomalus* M15 was inoculated to 5 mL of a YPD (glucose of 20 g/L, yeast extract of 10 g/L, and peptone of 20 g/L) liquid medium for culture at 30°C to a logarithmic growth phase. Total genomic DNA was extracted from the *Wickerhamomyces anomalus* by using a Yeast DNA Kit (purchased from Omega).

A pair of specific primers was designed for a sequence of a coding gene for a hypothetical protein derived from *Wickerhamomyces anomalus* NRRL Y-366-8 [it was named WICANDRAFT_92107 (NCBI accession number: XP_019039214.1), and included a total of 264 amino acids; and a nucleotide sequence of its coding gene (NCBI accession number: XM_019186339.1) included a total of 795 nucleotides]; besides, a base sequence homologous to a vector was introduced at a 5' end of each primer for construction of an YEp352 expression vector by a homologous recombinant clone method; and with 0.5 µL (about 10 ng) of the above total DNA solution as a template, and a PCR enzyme as KOD FX (purchased from TOYOBO, Japan), amplification was performed to obtain a target gene fragment, i.e. a gene fragment of oxidoreductase NRRL. After the target gene fragment was sequenced, its nucleotide sequence was shown as SEQ ID No. 1, and its amino acid sequence was shown as SEQ ID No. 2. Through comparison on the sequences in NCBI, it was found that the target gene fragment had a homology of 100% with the above hypothetical protein from *Wickerhamomyces anomalus* NRRL Y-366-8.

Herein, specific amplification primers were as follows (underlined marker sequences were bases homologous to a sequence of the vector):
NRRL-F: 5'-AGTTTCGAATAAACACACATAAACAAACAAAATGACTTTAAACACCCAAA-3 ; and
NRRL-R: 5'-GACCAAACCTCTGGCGAAGAAGTCCAAAGCTTTAATGATACATTATTCCA-3'.

**Table 1. KOD-FX PCR system and conditions**

| Reagent | Dose (µL) | | Reaction condition | |
|---|---|---|---|---|
| KOD FX | 0.5 | 1 | 95°C | 5 min |
| 2 × KOD Buffer | 12.5 | 2 | 95°C | 30 s |
| 5 × dNTP | 2 | 3 | 55°C | 15 s |
| NRRL-F | 1 | 4 | 72°C | 1 min |
| NRRL-R | 1 | 5 | Go to 2 for 30 times more | |
| Template | 1.25 | 6 | 72°C | 5 min |
| ddH₂O | 3.75 | 7 | 16°C | forever |
| Up to | 25 | | | |

After a PCR reaction, whether a size of a gene fragment is correct was detected by agarose gel electrophoresis; the amplified fragment was purified by using an oligonucleotide purification kit (purchased from Omega); a concentration of the gene fragment was detected by using a microspectrophotometer; and amplification was performed to obtain the target gene fragment, i.e. the gene fragment of the oxidoreductase NRRL.

A genome of *S. cerevisiae* CEN. PK2-1 Ca was extracted by using the Yeast DNA Kit. With the genome as a template, a TDH3 promoter fragment was amplified by a TDH3-F/TDH3-R primer pair, and an ADH1 terminator fragment was amplified by an ADH1-F/ADH1-R primer pair.

Sequences of the used primers were as follows (underlined marker sequences were homologous bases):
TDH3-F: 5'-TGACCATGATTACGAATTCTTTACCGTCGACACAGTTTATTCCTGGCATC-3';
TDH3-R: 5'-GACCAAACCTCTGGCGAAGAAGTCCAAAGCTTTTGTTTGTTTATGTGTGT-3';
ADH1-F: 5'-GAATAAACACACATAAACAAACAAAAGCTTTGGACTTCTTCGCCAGAG-3; and
ADH1-R: 5'-TTGCATGCCTGCAGGTCGACTCTAGAGGATCCATAGGGTAGGGGAATTTC-3'.

An YEp352 plasmid vector fragment purchased from Invitrogen was amplified by an YEp352-F1/YEp352-R1 primer pair.
YEp352-F1: 5'-GATCCTCTAGAGTCGACCTGCAGG-3'; and
YEp352-R1: 5'-GTCGACGGTAAAGAATTCGTAATCAT-3'.

The obtained TDH3 promoter fragment, ADH1 terminator fragment and YEp352 plasmid vector fragment were subjected to multi-fragment homologous recombination ligation by using a ClonExpress II recombinant clone kit (purchased from Vazyme Biotech Co., Ltd., Nanjing) to obtain the vector YEp352-TDH3ₚᵣₒₘₒₜₑᵣ-ADH1ₜₑᵣₘᵢₙₐₜₒᵣ, referred to as YEp352-TDH3ₚ-ADH1ₜ.

YEp352-TDH3ₚ-ADH1ₜ was amplified with YEp352-F2 (5'-AGCTTTGGACTTCTTCGCC-3') and YEp352-R2 (5'-TTTGTTTGTTTATGTGTGTT-3'), and then, the vector fragment amplified by YEp352-F2/R2 and the target gene obtained above were subjected to homologous recombination ligation by using the ClonExpress II recombinant clone kit (purchased from Vazyme Biotech Co., Ltd., Nanjing).

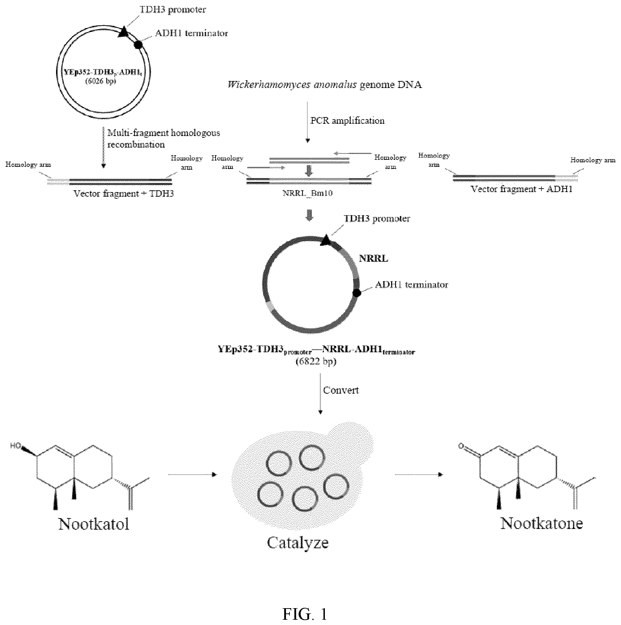

Then, 10 µL of a ligation product was chemically transformed into an *E*. *coli* DH5α competent cell, and coated on an LB/Amp (10 g/L tryptone, 5 g/L yeast extract, 5 g/L NaCl, and 15 g/L agar, autoclaving was performed at 121°C for 20 min; and Ampicillin with a final concentration of 1000 mg/mL was added before use) plate for culture at 37°C for 12-16 h to obtain a recombinant strain *E. coli* (YEₚ352-TDH3ₚ-NRRL-ADH1ₜ). A construction map was shown as FIG. 1. The recombinant strain was selected to be inoculated to 5 mL of an LB liquid medium containing 100 µg/mL ampicillin (10 g/L tryptone, 5 g/L yeast extract, and 5 g/L NaCl, autoclaving was performed at 121°C for 20 min; and the Ampicillin with the final concentration of 1000 mg/mL was added before use) for culture at 37°C and 220 rpm for 12 h; and then, a plasmid was extracted by using a rapid plasmid extraction kit (purchased from Tiangen (Beijing) Co., Ltd.) and sent to Sangon Bioengineering (Shanghai) Co., Ltd. for gene sequencing. Sequencing results were analyzed by using Snapgene software, to obtain a recombinant plasmid YEₚ352-TDH3ₚ-NRRL-ADH1ₜ containing an oxidoreductase gene.

### Example 2: Construction of Recombinant Expression Cell

The recombinant expression plasmid YEₚ352-TDH3ₚ-NRRL-ADH1ₜ constructed in example 1 was transformed into the *S. cerevisiae* CEN. PK2-1 Ca competent cell by using a *S*. *c.* Easy Comp Transformation Kit, as a yeast transformation kit, (purchased from Invitrogen, USA).
1) 500 ng of the recombinant plasmid and 250 µL of Solution III (Transformation solution) were added to each 25 µL of competent cells;
2) a mixture was uniformly oscillated by a vortex oscillator, and placed in a constant temperature incubator at 30°C for 15 min;
3) a resultant was taken out for uniform oscillation again, and placed at 30°C for 15 min, and then, the operation was repeated twice; and
4) then, 200 µL of recombinant yeast cells were taken and uniformly coated on a nutrition-defective plate SD/ΔUra (6.7 g/L YNB, 2 g/L amino acid mixture, 20 g/L glucose, 20 mg/L leucine, and 20 mg/L tryptophan) for culture at 30°C for 2-4 d.

The recombinant expression cell *S. c.* CEN.PK2-1 Ca (YEp352-TDH3ₚ-NRRL-ADH1ₜ) was obtained until monoclonal cells were grown on the plate.

### Example 3: Preparation of Nootkatone with Whole-Cell in Vitro Catalysis

The recombinant expression cell *S. c.* CEN. PK2-1 Ca (YEp352-TDH3ₚ-NRRL-ADH1ₜ) obtained in example 2 was selected and placed in a test tube containing 5 mL of SD/ΔUra liquid medium for culture at 30°C and 220 rpm for 24 h. Then, a resultant was transferred to a 250 mL shake flask containing 50 mL of SD/ΔUra liquid medium at an inoculation amount of initial OD₆₀₀ of 0.05, and cultured at 30°C and 220 rpm for 24 h. Subsequently, a bacteria solution with a total OD600 of 50 was collected, and centrifuged at 3000 rpm and 4°C for 5 min, and a supernatant was discarded. Then, the recombinant expression cells were resuspended in a potassium phosphate buffer (50 mM, pH 7.4), and its volume was fixed at 1 mL to obtain a reaction solution of 50 OD₆₀₀/mL. 20 µL of 100 mM nootkatol solution (containing 1% (v/v) Triton-100, dissolved into dimethyl sulfoxide) was added to make a final concentration of a substrate be 2 mM, and catalyzing was performed at 25°C and 220 rpm for 24 h.

A product detection method was as follows (the product detection methods in the subsequent examples are the same as that in example 3):
after the reaction, the reaction solution was transferred to a 2 mL centrifuge tube, and 1 mL of ethyl acetate was added for shaking extraction for 10 min, followed by centrifugation at a maximum speed for 5 min. After an organic layer was separated from a water layer, 500 µL of upper ethyl acetate was taken and put into a 1.5 mL centrifuge tube, and then, 500 µL of ethyl acetate was added. Finally, a mixture was filtered with a 0.22 µm organic filter membrane into a chromatographic bottle for gas phase detection. A gas phase used was a Hewlett-Packard 5890 II gas chromatograph. A chromatographic column was a 5% Ph-Me siloxane column, and was of a specification of 30 m × 0.10 mm × 0.10 µm. A detector was a flame ionization detector (FID). A carrier gas was N₂.

A detection method was as follows: 1 µL of a sample was subjected to split stream sampling at a split ratio of 15: 1, a sample inlet temperature of 250°C and a detector temperature of 350°C; the column temperature was maintained at 100°C for 5 min, then raised to 200°C at 20°C/min, and maintained at 200°C for 5 min. A total time lasted for 15 min.

A gas phase used was a Hewlett-Packard 5890 II gas chromatograph. A chromatographic column was a quartz capillary, and was of a specification of 30 m × 0.25 mm × 0.25 µm. A detector was a mass selective detector (MSD). The program was consistent with the above. A mass scanning way is to select ion scanning. Mass spectrometry conditions: an electron impact (EI) ionization source, and an electron energy of 70 eV

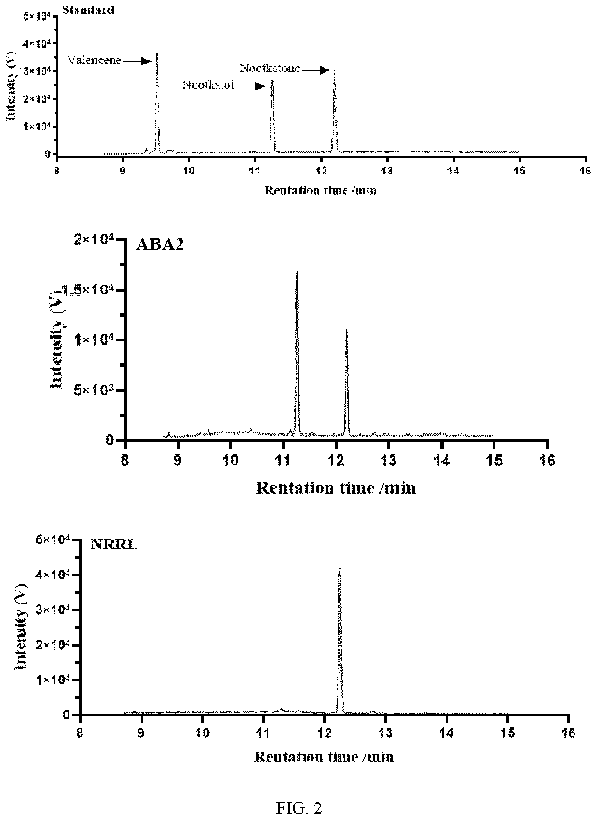

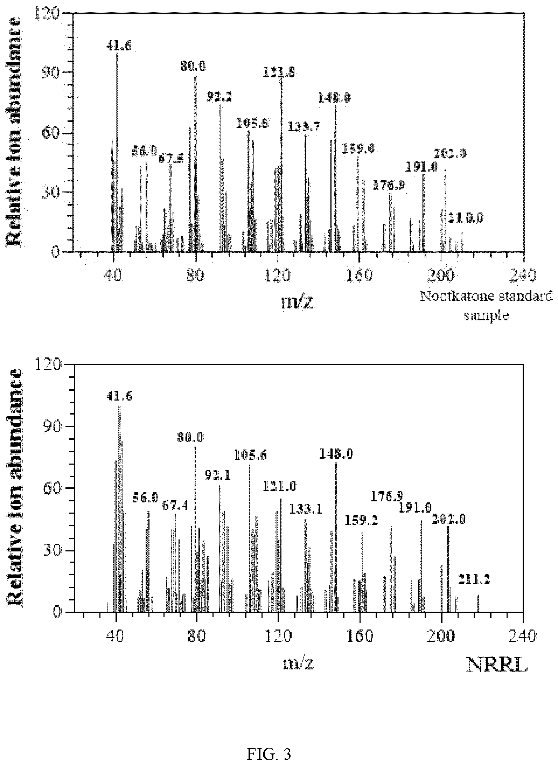

A standard substance was detected by GC-FID, and a retention time of nootkatone was detected to be 19.540 min. Gas phase detection results of whole-cell catalysis with some candidate alcohol dehydrogenases were shown in FIG. 2. A structure of a substance was further qualitatively analyzed by GC-MS, and results are shown in FIG. 3. A chromatogram and a mass spectrogram of the sample were compared with those of the standard substance respectively, which were consistent with the standard substance. Therefore, all the constructed recombinant yeast strains could successfully convert the valencene to produce the nootkatol and nootkatone.

Finally, a conversion rate of the recombinant expression cell *S. c.* CEN. PK2-1 Ca (YEp352-TDH3ₚ-NRRL-ADH1ₜ) of the oxidoreductase to the nootkatol was 100%.

Similarly, according to the methods described in examples 1 to -3, the present disclosure constructed a recombinant expression cell *S. c.* CEN. PK2-1 Ca (YEp352-TDH3ₚ-ABA2-ADH1t) of oxidoreductase ABA2 (NCBI accession number: HM036684.1) derived from citrus at the same time, and their catalytic ability under the same conditions was tested. Detection results were shown in FIG. 2, and results were 26% of the conversion effect described in example 3. The catalytic efficiency of the recombinant expression cell *S. c.* CEN. PK2-1 Ca (YEp352-TDH3ₚ-NRRL-ADH1ₜ) of the oxidoreductase of the present disclosure under the same conditions was 3.8 times that of the recombinant expression cell corresponding to ABA2, showing a good ability of the oxidoreductase of the present disclosure to convert the nootkatol.

### Example 4: Optimization on Oxidoreductase NRRL with Codon

The sequence of the oxidoreductase NRRL capable of catalyzing conversion of the nootkatol to nootkatone was evaluated according to a codon adaptable index (CAI) of *Saccharomyces cerevisiae,* and analyzed by the software from Detai Biotechnology Company (http://www.detaibio.com/). The sequence of the oxidoreductase NRRL with high catalytic performance and low CAI was entrusted to Sangon Bioengineering Co., Ltd. (Shanghai) for codon optimization and sequence synthesis. The sequence of the codon-optimized NRRL (cp) was constructed into an YEp352-TDH3ₚ-ADH1ₜ expression cassette according to the methods in example 1 and example 2, to obtain the YEp352-TDH3ₚ-NRRL(cp)-ADH1ₜ plasmid, and the recombinant expression cell *S. c*. CEN. PK2-1 Ca (YEp352-TDH3ₚ-NRRL(cp)-ADH1ₜ) was further obtained. A nucleotide sequence of NRRL (cp) was shown as SEQ ID No. 3.

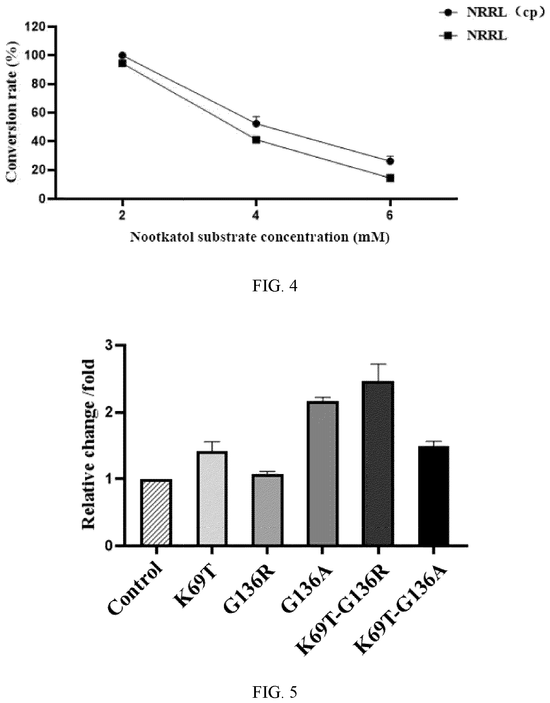

According to the method in example 3, a whole-cell catalysis experiment was performed on a strain having an original sequence and the optimized stain, to evaluate a difference in catalytic performance between the non-optimized strain and the optimized strain. Results were shown in FIG. 4. When a substrate concentration was increased, conversion rates of all the strains to the substrate had a decreasing trend; however, the conversion rate of the codon-optimized NRRL (cp) strain had a slighter decrease. When the substrate concentration was 4 mM or 6 mM, the conversion rate was increased by 1.26 times compared with the original strain. It was indicated that after codon optimization, an expression level of NRRL (cp) in a host bacterium could be increased to a certain extent, so as to further improve its catalytic performance.

### Example 5: Preparation of Mutant of Oxidoreductase NRRL

Through simulation analysis on a protein spatial structure of the oxidoreductase NRRL, its mutation sites were determined as follows: lysine K at the 69ₜₕ site was mutated to threonine T, and glycine G at the 136ₜₕ site was mutated to arginine R or alanine A.

Mutation primers with site-directed mutation were designed according to the NRRL optimized sequence SEQ ID NO. 3 (NRRL (cp)); and the gene of the oxidoreductase NRRL was mutated by a site-directed mutation method for a gene, so as to obtain a new oxidoreductase gene. The recombinant plasmid YEp352-TDH3ₚ-NRRL(cp)-ADH1t was used as a template, and a rapid PCR technique was adopted. Universal primers for the recombinant plasmid were as follows:
YEp352-F3: 5'-GTGACCGTCTCCGGGAGCTGCATGTG-3'; and
YEp3 52-R3 : 5'-CCGGAGACGGTCACAGCTTGTCTGTA-3'.

Single mutation was introduced at the 69ₜₕ site, and the primers were as follows (the underlined marker sequences were mutant bases):
NRRL (cp): K69T-F: 5'-CTTCTACGAAGCAAGAAATTTTCGAT-3';
NRRL (cp): K69T-R: 5'-CTTGCTTCGTAGAAGAATCACATGGA-3';
NRRL (cp): G136R-F: 5'-AATTGCGTAACCAACCAGGTAAAATT-3';
NRRL(cp): G136R-R: 5'-TGGTTACGCAATTCTTCGAACTTCT-3';
NRRL (cp): G136A-F: 5'-AATTGGCTAACCAACCAGGTAAAATT-3'; and
NRRL (cp): G136A-R: 5'-TGGTTAGCCAATTCTTCGAACTTCT-3'.

The above primers and the universal primers YEp352-F3/YEp352-R3 were amplified respectively. Amplification system and conditions were shown in Table 2:

**Table 2. PCR Amplification system and conditions**

| Reagent | Dose (µL) | | Reaction condition | |
|---|---|---|---|---|
| PrimeSTAR Max Premix | 25 | 1 | 94°C | 2 min |
| F1 | 0.5 | 2 | 98°C | 10 s |
| R1 | 0.5 | 3 | 52°C | 35 s |
| Template | 0.2 | 4 | 72°C | 1 min |
| ddH₂O | 23.8 | 5 | Go to 2 for 29 times more | |
| Up to | 50 | 6 | 72°C | 10 min |
| | | 7 | 16°C | forever |

After a PCR reaction, whether a size of a gene fragment is correct was detected by agarose gel electrophoresis; the amplified fragment was purified by using the oligonucleotide purification kit; and a concentration of the gene fragment was detected by using the microspectrophotometer to obtain DNA fragments of NRRL (cp): K69T-F/R, NRRL(cp): G136R-F/R and NRRL(cp): G136A-F/R.

By using the method in example 1, three kinds of recombinant plasmids containing single-site mutated genes of the oxidoreductase were obtained: YEp352-TDH3ₚ-NRRL (cp)-K69T/G136R/G136A-ADH1ₜ.

### Example 6: Preparation of Double-Site Mutant of Oxidoreductase NRRL

With the recombinant plasmid YEp352-TDH3ₚ-NRRL(cp)-K69T-ADH1ₜ constructed in example 5 as a template, a single mutation was introduced at the 136ₜₕ site to construct a double-site mutant. Primers were shown as NRRL (cp): G136R-F/R and NRRL (cp): G136A-F/R.

By using the methods in example 1 and example 5, two kinds of recombinant plasmids containing the double-site mutated oxidoreductase were obtained: YEp352-TDH3ₚ-NRRL (cp)-K69T-G136R/K69T-G136A-ADH1ₜ.

### Example 7: Catalytic Efficiency and Substrate Tolerance of Oxidoreductase Mutant

The five kinds of constructed recombinant expression vectors were transformed into the *S. c.* CEN. PK2-1 Ca competent cells by using the method of constructing the recombinant expression cell in example 2, so as to obtain five kinds of recombinant expression cells. Then, the whole-cell in vitro catalytic method in example 3 was used to test the conversion efficiency of the corresponding oxidoreductase mutant.

Results were shown in FIG. 5. The oxidoreductase mutant was significantly improved in vitro nootkatol conversion efficiency and substrate tolerance compared with the original oxidoreductase NNRL (cp). Beneficial mutation sites were: NRRL (cp): K69T, NRRL (cp): G136R, NRRL (cp): G136A, NRRL (cp): K69T-G136R and NRRL (cp): K69T-G136A. Compared with the original oxidoreductase NRRL (cp) in the same experimental system, the oxidoreductase conversion efficiency of the above beneficial mutant was 107% (such as NRRL (cp): G136R) to 247% (such as NRRL (cp): K69T-G136R). For many tested oxidoreductase mutants, such improvement on conversion efficiency usually occurred together with improvement on substrate tolerance. Results were shown in Table 3. Therefore, it was pointed out that the improvement on conversion efficiency did not depend on increased expressions of these mutants in the *Saccharomyces cerevisiae.*

**Table 3: Summary of substrate tolerance and conversion efficiency of modified oxidoreductases compared with SEQ ID NO. 2**

| Position^{a} | Original amino acid | Mutant amino acid | Substrate concentration (mM)^{b} | Conversion efficiency (% relative to wild type) ^{c} | Substrate tolerance (mM)^{d} |
|---|---|---|---|---|---|
| WT^{e} | - | - | 4 | 100 | 4 |
| K69T | K(AAG) | T(ACG)^{f} | 4 | 141 | 6 |
| G136R | G(GGA) | R(CGA) | 4 | 107 | 6 |
| G136A | G(GGA) | A(GCA) | 4 | 217 | 6 |
| K69T-G136R | K(AAG) & G(GGA) | T(ACG) & R(CGA) | 4 | 247 | 8 |
| K69T-G136A | K(AAG) & G(GGA) | T(ACG) & A(GCA) | 4 | 149 | 4 |

| | | | | | |
|---|---|---|---|---|---|
| ^{a}: a position of a modified oxidoreductase amino acid residue; and a residue coding started from an N-terminal threonine residue (= Met-1) in SEQ ID NO. 2. ^{b}: the test method referred to example 3, having a difference of different concentrations of the invested substrate for the nootkatol added. ^{c}: the conversion efficiency of the modified oxidoreductase (= sample) relative to the conversion efficiency obtained by using wild-type oxidoreductase (= control), i.e. {(Nootkatone [sample]/substrate concentration [sample])/(Nootkatone [control]/substrate concentration [control]) × 100%. ^{d}: the tolerance concentration of the enzyme which could still maintain a conversion rate of more than 60% under the test conditions. ^{e}: oxidoreductase wild type (SEQ ID NO. 2). ^{f}: the contents in the brackets () represent the codons used when the corresponding amino acids were mutated. | | | | | |

### Example 8: Salt Tolerance of Oxidoreductase Mutant

According to the whole-cell in vitro catalytic method in example 3, 3%, 6%, 9%, 12%, 15%, and 18% NaCl (W/V, g/100 mL) were added to the reaction system for catalysis at 25 °C and 220 rpm for 24 h. The effect of the oxidoreductase mutant on the substrate conversion rate was researched.

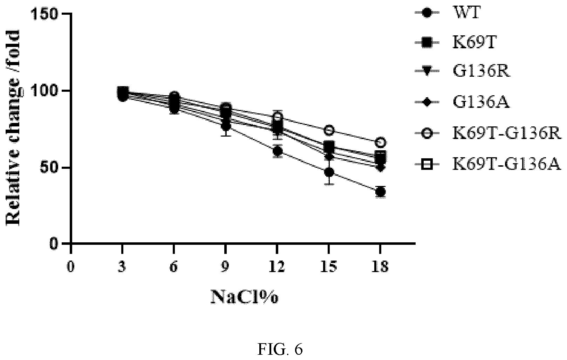

Results were shown in FIG. 6, WT (unmutated NRRL) was catalyzed in the 3% NaCl (W/V) reaction system for 24 h, and the substrate conversion rate was not affected. After treatment in 6%, 9%, 12%, 15%, and 18% NaCl (W/V) for 24 h, for the above five mutants with higher substrate catalytic efficiency and tolerance: NRRL (cp): K69T, NRRL (cp): G136R, NRRL (cp): G136A, NRRL (cp): K69T-G136R, and NRRL (cp): K69T-G136A, with the increase in the concentration of NaCl (W/V), the substrate conversion rates had a decreasing trend, but the substrate conversion rates of the mutants were all higher than that of WT, indicating that the salt tolerance of each mutant was slightly improved compared with the WT.

Based on the above results, NRRL (cp): K69T-G136R was the optimal mutant. Through single-site or double-site mutation, the oxidoreductase mutants of the present disclosure had higher conversion efficiency, substrate tolerance and salt tolerance than the wild-type oxidoreductase, having a good industrial application prospect.

### Example 9: Preparation of Nootkatone with Simple Carbon Source by Using Biological Fermentation

Using the technology described in Patent with Application No. 201910271558.6, the host cell *S. c.* CEN. PK2-1 Ca has been genetically modified, including knocking out a limiting factor rox1 for a mevalonate pathway, and down-regulating the expression intensity of a downstream branch pathway-related enzyme erg9 of a precursor FPP for sesquiterpenoids, so as to increase a supply of the precursor FPP; and then, an *S. c.* PK2-M strain was finally obtained. The valencene synthase ValC gene (NCBI accession number: JX040471) (a promoter in an expression cassette of the gene was PDC1, and a terminator was SAG1) derived from *Chamaecyparis nootkatensis* described in International Patent with Application No. PCT/NL2010/050848 and a gene of *Saccharomyces cerevisiae* endogenous HMG-CoA reductase tHMG1 (a rate-limiting step enzyme for the mevalonate pathway) (NCBI accession number: NM_001182434) subjected to cutting of an N-terminal regulatory region (a promoter in an expression cassette of the gene was TEF1, and a terminator was CYC1) were introduced into the recombinant expression vector YEp352-TDH3ₚ-NRRL(cp)-ADH1ₜ obtained in embodiment 4; and the recombinant expression vector YEp352-ValC-tHMG1-NRRL (cp) was obtained. In addition, TEF1p-Cas9-CYC1t in a p414-TEF1p-Cas9-CYC1t vector (a commercial plasmid of Addgene) was replaced with a gene of cytochrome P450 monooxygenase HPO (NCBI accession number: EF569601) (a promoter in an expression cassette of the gene was HXT7, and a terminator was TPI1)derived from *Hyoscyamus muticus* described in Patent WO 2006/079020 and an AtCPR gene of cytochrome reductase (NCBI accession number: NM_118585) (a promoter in an expression cassette of the gene was HXT7, and a terminator was TPI1)from *Arabidopsis thaliana* described in the paper by Urban, Pd et al. in 1997 (Urban, P., et al. Cloning, yeast expression, and characterization of the coupling of two distantly related Arabidopsis thaliana NADPH-cytochrome P450 reductases with P450 CYP73A5. J. Biol. Chem. 1997, 272, 19176-19186.); and the recombinant expression vector p414-HPO-AtCPR was obtained. The recombinant expression vectors YEp352-ValC-tHMG1-NRRL (cp) and p414-HPO-AtCPR were transformed into the previously constructed high-yield FPP engineering strain S. c. PK2-M, to obtain recombinant expression cells S. c. PK2-M (YEp352-ValC-tHMG1-NRRL (cp) and p414-HPO-AtCPR), referred to as "PK2-VHN (cp)-HA".

The PK2-VHN (cp)-HA strain was inoculated to a test tube containing 5 mL of SD/ΔTrp-Ura medium (6.7 g/L YNB, 2 g/L amino acid mixture, 20 g/L glucose, and 20 mg/L leucine), and cultured on a shaker at 30°C and 220 rpm for about 20 h. After OD₆₀₀ of the bacteria solution reached 1-3, a resultant was transferred to a 50 mL conical flask containing 10 mL of SD/ΔTrp-Ura medium, covered with a 20% n-dodecane (2 mL) organic phase, and fermented on the shaker at 25°C and 220 rpm for 96 h.

After fermentation, 500 µL of the upper n-dodecane organic phase was taken and mixed with an equal volume of ethyl acetate. A product was detected according to the gas phase detection method in example 4.

In final fermentation results, a yield of nootkatone was 24 mg/L, accounting for 20% of a yield of total terpenoids (a sum of the valencenes, the nootkatol and nootkatone). If the used oxidoreductase was replaced with ABA2 derived from the citrus, the yield of nootkatone was only 2.5 mg/L, accounting for 2.6% of the yield of the total terpenoids. It was indicated that the fermentation yield of nootkatone could be increased by 9.6 times, and the purity could be increased by 12 times after the use of the oxidoreductase of the present disclosure. If the oxidoreductase mutants (such as NRRL (cp): K69T-G136R) were continued to be replaced for use, the yield of nootkatone was increased to 42 mg/L, being 1.75 times that of the wild-type oxidoreductase. It was proved that the oxidoreductase of the present disclosure had better catalytic performance than the previously reported isozymes, and also had higher tolerable substrate concentration. By using the oxidoreductase and the mutant thereof, a higher fermentation yield of nootkatone and a product with higher purity could be obtained.

The above embodiments are preferred implementations of the present disclosure, but the implementations of the present disclosure are not limited by above embodiments. Any other changes, modifications, substitutions, combinations and simplifications made without departing from the spirit and principle of the present disclosure shall be equivalent replacement methods, and fall within the scope of protection of the present disclosure.

## Claims

1. Use of oxidoreductase and a mutant thereof in biosynthesis of nootkatone, wherein
the oxidoreductase is named NRRL, and an amino acid sequence of the oxidoreductase is shown as SEQ ID No.2; and
the oxidoreductase mutant has an amino acid sequence obtained by mutating one or two sites of the 69ₜₕ and 136ₜₕ amino acids of SEQ ID No. 2.

2. The use according to claim 1, wherein
the oxidoreductase mutant is used for improving substrate tolerance, conversion rate, and salt tolerance, and a substrate is nootkatol.

3. The use according to claim 1, wherein
the oxidoreductase mutant has the amino acid sequence obtained by mutating one or two sites of the 69ₜₕ and 136ₜₕ amino acids of SEQ ID No. 2, the 69ₜₕ amino acid is mutated from lysine K to threonine T, and the 136ₜₕ amino acid is mutated from glycine G to arginine R or alanine A.

4. The use according to claim 3, wherein
the amino acid sequence of the oxidoreductase mutant is obtained by mutation of the 69ₜₕ amino acid of SEQ ID No. 2 from the lysine K to the threonine T and the 136ₜₕ amino acid of SEQ ID No. 2 from the glycine G to the arginine R, and the specific amino acid sequence is shown as SEQ ID No. 4.

5. The use according to any one of claims 1-4, wherein
in the oxidoreductase mutant, the sequence of a gene coding the amino acid sequence shown as SEQ ID No. 2 is shown as SEQ ID No. 3.

6. The use according to any one of claims 1-4, wherein
a nucleotide sequence of an oxidoreductase NRRL coding gene is shown as SEQ ID No.1 or SEQ ID No. 3.

7. An oxidoreductase mutant according to any one of claims 1-5.

8. A gene for coding the oxidoreductase mutant according to claim 7.

9. A recombinant expression vector or a recombinant expression cell containing the gene according to claim 8.

10. Use of the recombinant expression vector or the recombinant expression cell containing the coding gene according to claim 6, the recombinant expression vector or the recombinant expression cell according to claim 9 in biosynthesis of nootkatone.
